# EUROPEAN PATENT APPLICATION

(11) **EP 4 234 684 A1**
(43) Date of publication of application: **30.08.2023**
(21) Application number: 22159206.6
(22) Date of filing: 28.02.2022
(51) Int. Cl.: C12N 5/0783, A61P 35/00

(54) **MODIFIED IMMUNE CELLS FOR TREATING AND/OR PREVENTING METASTASIS**

(71) Applicant: Technische Universität München, 80333 München (DE)
(72) Inventor: Knolle, Percy, 81735 München (DE); Peddle, Anna-Marie, 80634 München (DE)
(74) Representative: Engelhard, Markus

(57) **Abstract**

The present invention relates to modified immune cells, in particular modified NK cells and T cells. The present invention relates to the modified NK cells and T cells for use in the treatment and/or prevention of cancer, in particular in the treatment and/or prevention of metastases. The present invention further relates to a method of generating modified NK cells or T cells. The present invention further relates to compositions comprising said modified immune cells.

## Description

The present invention relates to modified immune cells, in particular modified NK cells and T cells. The present invention relates to the modified NK cells and T cells for use in the treatment and/or prevention of cancer, in particular in the treatment and/or prevention of metastases. The present invention further relates to a method of generating modified NK cells or T cells. The present invention further relates to compositions comprising said modified immune cells.

### BACKGROUND OF THE INVENTION

Our immune system is uniquely able to detect the neoplastic transformation of host cells, i. e. cancer. Cancer immunosurveillance critically relies on the activity of so-called natural killer (NK) cells, which express a repertoire of immune receptors enabling them to recognize and eliminate cancer cells. Multiple studies have demonstrated that NK cells fulfill a central role in the elimination of cancer metastasis (Glasner et al., Immunity, 2018; Morvan and Lanier, Nature Reviews in Cancer, 2016), which is in part mediated by their ability to produce immunostimulatory cytokines and chemokines, such as IFNγ, TNF-alpha, XCL1 and CCL5.

Up to date, various strategies have been designed to exploit the potential of NK cells for treatment of metastatic disease (Bald et al., Nature Immunology, 2020; Lorenzo-Herrero et al, Cancers, 2019; Guillerey et al., Nature Immunology, 2016). Such strategies aim to enhance immunosurveillance of metastases by boosting NK cell proliferation and persistence or by stimulating activity and effector function of NK cells *in vivo.* However, metastatic cancer cells can develop multiple suppressive mechanisms that enable evasion of NK cell surveillance. Such suppressive mechanisms are currently thought to be the major reason why NK cell-based immunotherapies for treatment of metastasis show only limited success in the clinic. These strategies include the downregulation of molecules that confer tumor recognition and activation of NK cells, or the production of immunomodulatory molecules that directly or indirectly suppress NK cell functions.

One suppressive mediator that has been suggested to limit anti-metastatic immunity is the eicosanoid prostaglandin E₂ (PGE₂) (Morvan and Lanier, Nature Reviews in Cancer, 2016; Wang and DuBois, Trends Mol Med, 2016; Zelenay et al., Cell, 2015; Böttcher et al., Cell, 2018). Increased PGE₂ levels resulting from enzymatic activity of cyclooxygenases (COX) 1 and 2 within metastatic tissue have been found in various human malignancies including colon, breast and pancreatic cancer, and high PGE₂ levels are associated with poor prognosis of cancer patients with metastatic disease. Therapeutic strategies designed to block production of PGE₂ by the use of inhibitors such as steroids (inhibitors of Arachidonic acid release) and nonsteroid anti-inflammatory drugs (blockers of cyclooxygenases) have proven ineffective in limiting the level of PGE2 in tumor or metastasis tissue and failed to impact on patient survival (Burn et al., Lancet, 2020; Guo et al., Medicine, 2019), and by design cannot selectivity target inhibitory PGE2 signaling on key immune cells.

Recent studies indicated that PGE₂ can directly signal onto NK cells to suppress their activity within primary tumors, identifying these cells as a key cellular target of PGE₂ signaling in solid tumors (Böttcher et al., Cell 2018, Bonavita et al., Immunity 2020). However, the mechanisms how PGE₂ signaling regulates NK cell biology and function and its consequences for NK cell-mediated control of metastasis *in vivo* have hardly been investigated so far. It is unknown whether certain receptors expressed on NK cells can be blocked to disable PGE2-mediated suppression and thereby enable NK cells to eliminate metastases.

There is a need in the art for improved methods and means for the treatment and/or prevention of cancer and especially metastases.

### SUMMARY OF THE INVENTION

According to the present invention this object is solved by a modified natural killer (NK) cell or a modified T cell, wherein the expression and/or activity of prostaglandin E receptor 2 (EP2) and of prostaglandin E receptor 4 (EP4) is selectively inhibited or eliminated.

According to the present invention this object is solved by providing the modified NK or T cell of the present invention for use in the treatment and/or prevention of metastatic disease and cancer.

According to the present invention this object is solved by a method for generating a modified NK or T cell according to the present invention, comprising the following steps:
(a) providing leukocytes, preferably lymphocytes, from a healthy subject/donor or from a patient,
(b) separating the NK or T cells and *in vitro* expanding the NK or T cells;
(c) genetically modifying the NK or T cells in order to selectively eliminate the expression of EP2 and EP4;
   preferably by knocking out EP2 and EP4 or by gene silencing/knockdown of EP2 and EP4, and
(d) *in vitro* expanding the genetically modified NK or T cells ;
(e) optional, cryopreserving the genetically modified NK or T cells obtained in step (d).

According to the present invention this object is solved by a method for the treatment and/or prevention of cancer, preferably for the treatment and/or prevention of metastasis, comprising the step of
administering to a subject in need thereof modified NK and/or T cells,
wherein the expression and/or activity of prostaglandin E receptor 2 (EP2) and of prostaglandin E receptor 4 (EP4) is selectively inhibited or eliminated,
preferably administering modified NK and/or T cells according to the present invention or modified NK and/or T cells obtained by a method of the present invention.

According to the present invention this object is solved by a composition comprising
(a) a modified NK and/or T cell of the present invention,
   which are preferably obtained by a method of the present invention,
(b) optional, excipient(s) and/or carrier.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "1 to 21" should be interpreted to include not only the explicitly recited values of 1 to 21, but also include individual values and sub-ranges within the indicated range. Thus, included in this numerical range are individual values such as 1, 2, 3, 4, 5 .... 17, 18, 19, 20, 21 and sub-ranges such as from 2 to 10, 8 to 15, etc. This same principle applies to ranges reciting only one numerical value, such as "at least 90%". Furthermore, such an interpretation should apply regardless of the breadth of the range or the characteristics being described.

### Modified immune cells

As outlined above, the present invention provides a modified immune cell. In particular, the present invention provides a modified natural killer (NK) cell. In particular, the present invention provides a modified T cell.

In a modified NK or T cell of the present invention the expression and/or activity of prostaglandin E receptor 2 (EP2), which is encoded by the PTGER2 gene, and of prostaglandin E receptor 4 (EP4), which is encoded by the PTGER4 gene, is selectively inhibited or eliminated.

Preferably, the expression of prostaglandin E receptor 2 (EP2) and of prostaglandin E receptor 4 (EP4) is selectively inhibited or eliminated by genetic modification.

Prostaglandin E receptor 2, also known as EP2, is a prostaglandin receptor for prostaglandin E2 (PGE2) and is encoded by the human gene *PTGER2.* It is one of four identified PGE2 receptors, the others being EP1, EP3, and EP4, encoded by the human genes PTGER1, PTGER3 and PTGER4, respectively, which bind with and mediate cellular responses to PGE2 and also, but with lesser affinity and responsiveness, certain other prostanoids.

The EP2 and EP4 prostanoid receptors are two of the four subtypes of receptors for prostaglandin E₂ (PGE2). They are in the family of G-protein coupled receptors and both receptors were initially characterized as coupling to Gs and increasing intracellular cAMP formation. The signalling through the two G_{S}- coupled receptors EP2 and EP4 activates the adenylate cyclase-triggered cAMP/PKA/CREB pathway, which mediates the dominant aspects of the anti-inflammatory and suppressive activity of PGE2 in immune cells. Signaling by both receptors can further activate the β-catenin/T-cell factor (Tcf) pathway, thereby additionally regulating gene transcription. The EP2 receptor does this primarily through cAMP-dependent protein kinase (PKA), whereas the EP4 utilizes phosphatidylinositol 3-kinase (PI3K) as well as PKA. In addition, the EP4 receptor can activate the extracellular signal-regulated kinases (ERKs) 1 and 2 by way of PI3K leading to the induction of early growth response factor-1 (EGR-1), a transcription factor traditionally associated with wound healing. This induction of EGR-1 expression has significant implications concerning the potential role of PGE₂ in cancer and inflammatory disorders.

In one embodiment, the modified NK or T cell of the present invention is genetically modified by knocking out EP2 and EP4,
more preferably by using the CRISPR/Cas9 gene editing system.

In one embodiment, the modified NK or T cell of the present invention is modified or edited by knocking out EP2 and EP4,
more preferably by using zinc-finger nucleases (ZFNs), transcription activator-like effector nucleases (TALENs) and MegaTAL nucleases, or by using base editing.

In one embodiment, the modified NK or T cell of the present invention is modified by gene silencing/knockdown of EP2 and EP4,
more preferably via introduction of short-hairpin RNAs via the Sleeping Beauty transposon system, lentiviral vectors or gamma-retroviral vectors.

In a preferred embodiment, the modified NK cell of the present invention is a so-called SUPER NK cell.

Wherein SUPER refers to SUPpression by EP2/EP4 Refractory NK cells. SUPER NK cells are modified so that they show reduced expression or decreased functionality of the PGE2 receptor EP2 and EP4, e.g. by a knockout of the EP2/EP4 encoding genes using CRISPR/Cas9 gene editing system. These SUPER NK cells are inert against their inhibition by PGE2 in metastases and maintain their full functional potential and anti-metastatic activity following transfer.

In one embodiment, the modified NK or T cell of the present invention are further modified. For example, the modified NK or T cell can be further modified, such as to enhance their function, such as by introducing component(s), e.g. a chimeric antigen receptor (CAR), a chemokine or cytokine receptor; an activating receptor; or by eliminating other genes, such as other inhibitory receptors.

### Medical uses of the modified immune cells

As outlined above, the present invention provides the modified NK or T cell of the present invention for use in the treatment and/or prevention of metastatic disease and cancer.

Preferably, the modified NK or T cell of the present invention is provided for use in the treatment and/or prevention of metastasis.

Preferably, the cancer is
- a solid tumor, preferably a primary or secondary solid tumor,
   such as, but not limited to melanoma, breast cancer, colorectal cancer, pancreatic cancer;
- a hematological tumor or malignancy,
   such as acute myeloid leukemia, multiple myeloma, non-Hodgkin lymphoma, Hodgkin lymphoma, B and T cell lymphoblastic leukemia;
and/or
- metastases,
preferably metastases in organs such as lung, liver, brain, bone marrow or lymphoid tissue.

Preferably, the treatment and/or prevention of cancer comprises adoptive immune cell therapy, preferably adoptive NK cell therapy, adoptive T cell therapy, CAR T cell therapy, CAR NK cell therapy and combinations thereof.

"Adoptive immune cell therapy" or "adoptive cell therapy (ACT)" refers to a form of therapy in which immune cells are transferred to tumor-bearing hosts. The immune cells have antitumor reactivity and can mediate direct or indirect antitumor effects.

In one embodiment, the treatment and/or prevention of cancer comprises an adjuvant therapy, such as after surgery of the primary tumor.

In one embodiment, the treatment and/or prevention of cancer comprises treatment of metastasis, including advanced metastasis.

In one embodiment, the treatment and/or prevention of cancer comprises a combination therapy with other immune therapies, such as checkpoint blockade inhibitors targeting PD-1, CTLA-4, and/or PD-L1.

In one embodiment, the treatment and/or prevention of cancer comprises a combination therapy with anti-programmed death (PD) protein 1 therapy.

In one embodiment, the treatment and/or prevention of cancer comprises combinations with with other immune therapies and with anti-programmed death (PD) protein 1 therapy.

### Methods for generating the modified NK or T cells

As outlined above, the present invention provides a method for generating a modified NK or T cell according to the present invention.

Said method comprises the following steps:
(a) providing leukocytes from a healthy subject/donor or from a patient;
(b) separating the NK or T cells and *in vitro* expanding the NK or T cells;
(c) genetically modifying the NK or T cells in order to selectively eliminate the expression of EP2 and EP4; and
(d) *in vitro* expanding the genetically modified NK or T cells;
(e) optional, cryopreserving the genetically modified NK or T cells obtained in step (d).

### Step (a)

In step (a) of the method of the present invention, leukocytes are provided.

Preferably, the leukocytes are lymphocytes.

Preferably the leukocytes (preferably lymphocytes), are
from periphery blood, preferably obtained via leukapheresis,
from cord blood, such as from a cord blood bank, or
from induced pluripotent stem cells.

In one embodiment, the leukocytes, preferably lymphocytes, are from autologous cells derived from a patient.

In one embodiment, the leukocytes, preferably lymphocytes, are allogeneic cells derived from an unrelated healthy subject/donor.

### Step (b)

In step (b) of the method of the present invention, the NK or T cells are separated from said leukocytes, preferably lymphocytes, and expanded *in vitro.*

Optionally, the NK or T cells can be cryopreserved before carrying out step (c).

### Step (c)

In step (c) of the method of the present invention, the NK or T cells are genetically modified in order to selectively eliminate the expression of EP2 and EP4.

The genetic modification is preferably by knocking out EP2 and EP4 or by gene silencing/knockdown of EP2 and EP4.

Preferably, the genetic modification is carried out by
(i) using the CRISPR/Cas9 gene editing system, or
(ii) using zinc-finger nucleases (ZFNs), transcription activator-like effector nucleases (TALENs) and MegaTAL nucleases, or
   by using base editing, or
(iii) gene silencing/knockdown via introduction of short-hairpin RNAs via the Sleeping Beauty transposon system, lentiviral vectors or gamma-retroviral vectors.

### Step (d)

In step (d) of the method of the present invention, the genetically modified NK or T cells are expanded *in vitro.*

### Step (e)

In step (e) of the method of the present invention, which is an optional step, the genetically modified NK or T cells obtained in step (d) are cryopreserved.

Cryopreserved cells are cells that have been preserved by cooling to a sub-zero temperature (degree Celsius).

These cells may or may not be preserved in the presence of a cryoprotective agent (such as DMSO, glycerol, ethylene glycol or propylene glycol, or other) that protects cells from damage associated with storage at sub-zero temperature/freezing such as membrane damage or ice crystal formation

In one embodiment, the NK or T cells can be further modified. The method of the invention comprises the optional step of additional modification of the NK cell or T cell, such as to enhance their function.

Examples for such further or additional modification are
introducing component(s), such as a chimeric antigen receptor (CAR), a chemokine or cytokine receptor; an activating receptor;
   or
the elimination of other genes, such as other inhibitory receptors.

### Methods of treatment

As outlined above, the present invention provides a method for the treatment and/or prevention of cancer, preferably for the treatment and/or prevention of metastasis.

Said method comprises the step of
administering to a subject in need thereof modified natural killer (NK) and/or T cells,
wherein the expression and/or activity of prostaglandin E receptor 2 (EP2) and of prostaglandin E receptor 4 (EP4) is selectively inhibited or eliminated.

Preferably, modified NK and/or T cells according to the present invention or modified NK and/or T cells obtained by a method of the present invention are administered.

In a preferred embodiment, the method for the treatment and/or prevention of cancer comprises the following steps:
(a) providing leukocytes from a healthy subject/donor or from a patient,
(b) separating the NK or T cells and *in vitro* expanding the NK or T cells;
(c) genetically modifying the NK cells in order to selectively eliminate the expression of EP2 and EP4;
(d) *in vitro* expanding the genetically modified NK or T cells ;
(e) optional, cryopreserving the genetically modified NK or T cells obtained in step (d);
(f) administering the genetically modified NK and/or T cells obtained in step (d) or from step (e), preferably via infusion.

### Step (a)

In step (a) of the method of the present invention, leukocytes are provided.

Preferably, the leukocytes are lymphocytes.

Preferably the leukocytes (preferably lymphocytes), are
from periphery blood, preferably obtained via leukapheresis,
from cord blood, such as from a cord blood bank, or
from induced pluripotent stem cells.

In one embodiment, the leukocytes, preferably lymphocytes, are from autologous cells derived from a patient.

In one embodiment, the leukocytes, preferably lymphocytes, are allogeneic cells derived from an unrelated healthy subject/donor.

### Step (b)

In step (b) of the method of the present invention, the NK or T cells are separated from said leukocytes, preferably lymphocytes, and expanded *in vitro.*

Optionally, the NK or T cells can be cryopreserved before carrying out step (c).

### Step (c)

In step (c) of the method of the present invention, the NK or T cells are genetically modified in order to selectively eliminate the expression of EP2 and EP4.

The genetic modification is preferably by knocking out EP2 and EP4 or by gene silencing/knockdown of EP2 and EP4.

Preferably, the genetic modification is carried out by
(i) using the CRISPR/Cas9 gene editing system, or
(ii) using zinc-finger nucleases (ZFNs), transcription activator-like effector nucleases (TALENs) and MegaTAL nucleases, or
   by using base editing, or
(iii) gene silencing/knockdown via introduction of short-hairpin RNAs via the Sleeping Beauty transposon system, lentiviral vectors or gamma-retroviral vectors.

### Step (d)

In step (d) of the method of the present invention, the genetically modified NK or T cells are expanded *in vitro.*

### Step (e)

In step (e) of the method of the present invention, which is an optional step, the genetically modified NK or T cells obtained in step (d) are cryopreserved.

### Step (f)

In step (f) of the method of the present invention, the genetically modified NK and/or T cells obtained in step (d) or from step (e) are administered.

Preferably, the administration is via infusion or via intra-tumoral or local injection,

### Compositions

As outlined above, the present invention provides a composition comprising
(a) a modified NK and/or T cell of the present invention, and
(b) optional, excipient(s) and/or carrier.

Preferably, the modified NK or T cell according to the present invention is obtained by a method of the present invention.

Preferably, the modified NK or T cells are cryopreserved.

Cryopreserved cells are cells that have been preserved by cooling to a sub-zero temperature (degree Celsius).

These cells may or may not be preserved in the presence of a cryoprotective agent (such as DMSO, glycerol, ethylene glycol or propylene glycol, or other) that protects cells from damage associated with storage at sub-zero temperature/freezing such as membrane damage or ice crystal formation

Suitable excipient(s) and/or carrier are:
- basal cell culture medium,
- isotonic solution,
   ∘ such as Plasmalyte
- serum albumin
- proteins, nucleic acids or other components supporting the growth, survival or activation of the cells,
   ∘ such as growth factors and cytokines, targeting antibodies, metabolites and nutrients.

### Preferred embodiments

### - Results

### 1) Effect of direct PGE₂-signaling on NK cells and their immunological function

As a first step, we characterized the PGE₂-mediated effects on NK cell function. To address this, conventional NK cells were isolated from the spleen of wildtype (WT) mice via cell sorting (for details, see Examples section) and cultured in low doses of IL2 and IL15/IL15Ra for five days. This *in vitro* cytokine culture allowed us to generate potent effector cells for the different assays.

NK cells are critical in anti-tumor immunity due to their ability to rapidly kill tumor cells via the secretion of cytotoxic granules. To assess whether pre-treatment with PGE₂ affects cytotoxicity of NK cells, degranulation and cytotoxicity assays were performed. Following PGE₂ treatment overnight, the ability of NK cells to degranulate upon stimulation was analyzed.

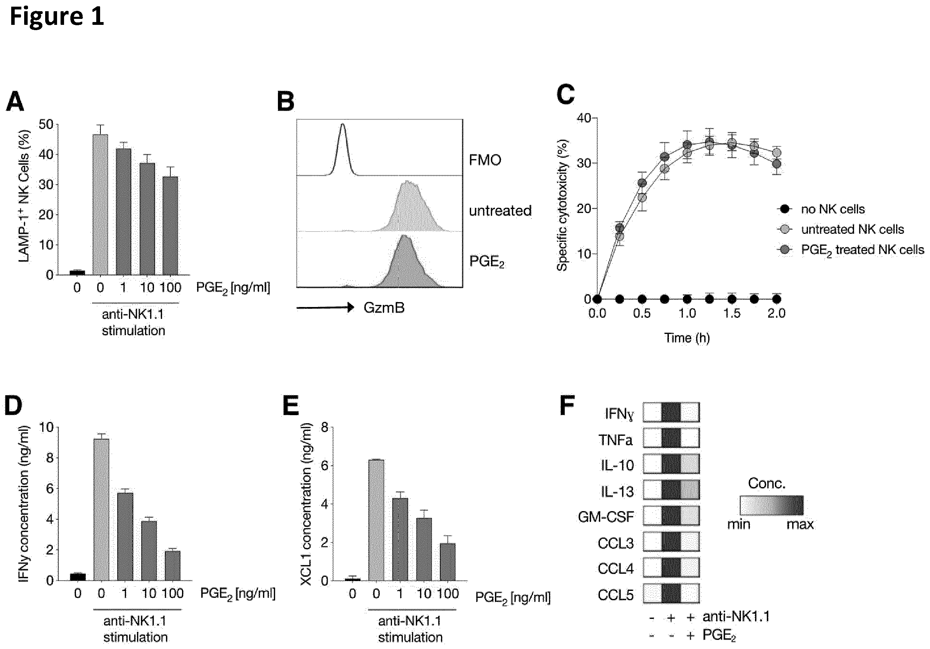

Different stimulation methods were used, such as triggering of activating receptors (NK1.1 or NKp46), addition of PMA and ionomycin or co-culture with tumor cells. The release of cytotoxic granules was assessed by flow cytometric measurement of LAMP-1 staining. Stimulation of NK cells with anti-NK1.1 induced strong degranulation (indicated by high LAMP-1 staining), which was only slightly reduced by PGE₂ (Figure 1A). Also, the granzyme B levels were unaffected when comparing the expression levels in untreated and PGE₂-treated NK cells (Figure 1B). These results indicate that PGE₂ does not impair NK cell degranulation and thus, their ability to kill tumor cells. To confirm these findings, we directly analyzed the killing potential of NK cells by real-time impedance measurements of tumor cells upon co-culture with NK cells via XCelligence DP system (Agilent Technologies Inc., USA). Both, untreated and PGE₂-treated cells effectively eliminate the tumor cells (Figure 1C). Besides their cytotoxicity, NK cells play an important immunoregulatory role in anti-tumor immunity. Upon stimulation, NK cells secrete an array of cytokines, chemokines and growth factors which orchestrate multi-immune responses. The impact of PGE₂ on the ability of NK cells to secrete cytokines and chemokines was analyzed following stimulation (stimulation methods as described in the Examples section) using quantitative multiplex assays and ELISA. Anti-NK1.1 induced strong IFNγ and XCL1 release, which was markedly reduced in a dose-dependent manner in presence of PGE₂ (Figure ID and E). Besides IFNγ and XCL1, PGE₂ signaling on NK cells diminished the secretion of various other effector cytokines, chemokines and growth factors upon stimulation (Figure F).

Taken together, the findings show that PGE₂ does not impair cytotoxicity but the secretion of cytokines and chemokines by NK cells. Thus, these cells were unable to use key intercellular communication pathways upon activation.

### 2) PGE₂-signaling stably imprints a dysfunctional program in NK cells

To determine the impact of PGE₂ signaling on the transcriptome of NK cells and to identify key regulated pathways, we performed RNA sequencing of NK cells.

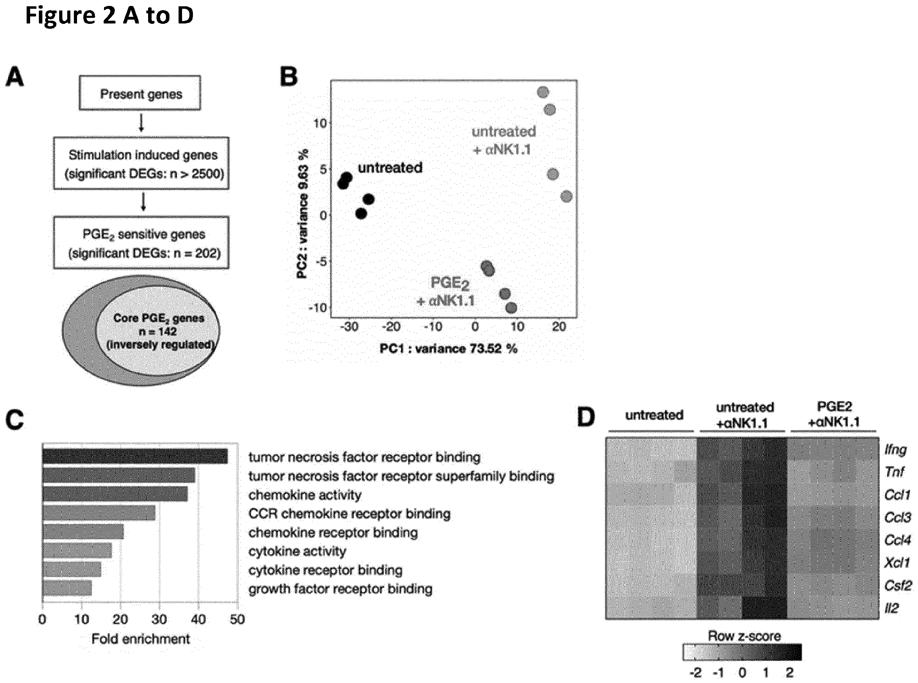

For this approach, NK cells were pre-treated with PGE₂ for 1 h and then stimulated with anti-NK1.1 for further 4 h. The data were analyzed and filtered according a specific scheme (Figure 2A). By comparing the gene expression pattern between unstimulated (untreated) and stimulated (untreated+aNK1.1), we first identified all genes that were upregulated or downregulated upon stimulation. We next analyzed the expression of these stimulation-induced genes in PGE₂-treated cells (PGE₂+aNK1.1). In the principal component analysis (PCA), we see a clear separation of untreated+aNK1.1 and PGE₂+aNK1.1 cells demonstrating that PGE₂ signaling affects the transcriptional program normally induced by anti-NK1.1 activation (Figure 2B). We then further focused on the differences in gene expression and identified 142 stimulation-induced genes that were inversely regulated upon PGE₂ pre-treatment (the so-called Core PGE₂ genes). We then filtered for those genes whose expression was hampered due to PGE₂ treatment and performed a pathway analysis. The majority of genes belong to cytokine, chemokine and growth factor pathways (Figure 2C). The heatmap showed exemplary effector cytokines, chemokines and growth factors that failed to be expressed upon stimulation due to PGE₂ treatment (Figure 2D).

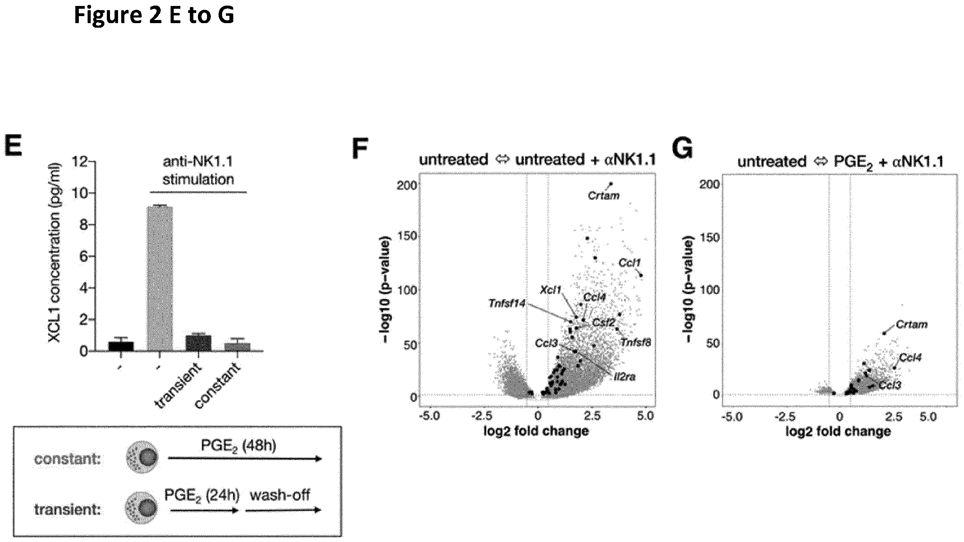

Next, we were interested in whether these immunosuppressive effects of PGE₂ signaling are reversible or irreversible. Therefore, we treated NK cells either constantly (48 h) or transiently (24 h then wash-off) with PGE₂ before stimulation with anti-NK1.1. Surprisingly, we still saw strong inhibitory effects on XCL1 secretion when we removed PGE₂ for 24 h (Figure 2E). These results gave us a first hint that the transcriptional changes mediated by PGE₂ signaling are stably imprinted. To confirm this, we employed an assay for transposase-accessible chromatin (ATAC) using sequencing. This technique allowed us to identify genomic regions whose accessibility is changed upon PGE₂ treatment. For this approach, NK cells were treated as in the RNA sequencing experiment described above. First, we looked for genomic regions that normally open upon stimulation. As depicted in the volcano plot of Figure 2F), promotor regions of effector cytokines and chemokines gained accessibility when NK cells were stimulated. In contrast, these regions failed to open in PGE₂-treated NK cells (Figure 2G). We conclude from these results that the PGE₂-mediated NK cell dysfunctionality is indeed epigenetically imprinted.

In summary, these experiments provided a global overview about the NK cell intrinsic pathways targeted by PGE₂ signaling and showed that PGE₂ signaling on NK cells stably imprints a dysfunctional program characterized by a selective block in transcriptional regulation of effector cytokines and chemokines.

### 3) PGE₂-mediated impairment of cytokine production in NK cells depends on the PGE₂ receptors EP₂ and EP₄

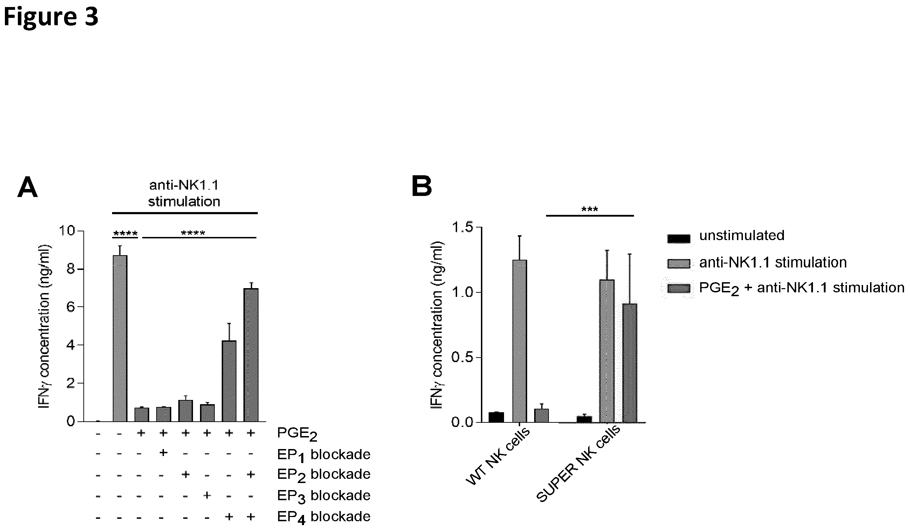

PGE₂ exerts its biological function by signaling via four different G-protein-coupled receptors (EP₁ to EP₄), which are linked to different intracellular signaling pathways. Specific inhibitors against the four PGE₂ receptors were used to determine receptor specificity of observed PGE₂ mediated effects. Blockade of EP₂ and EP₄ signaling almost reversed PGE₂ induced impairment of cytokine production (Figure 3A). Thus, EP₂ and EP₄ mediated the immunosuppressive signaling of PGE₂ on NK cells.

We underscored our findings by the use of NK cells that are deficient in EP₂ and EP₄, the so-called SUPER NK cells. These cells were isolated from GzmB-Cre Ptger2/ Ptger4fl/fl mice, in which only GzmB-expressing cells such as NK cells are selectively deficient in EP₂ and EP₄. Functional assays with the SUPER NK cells demonstrated that they are completely rescued from the inhibitory action of PGE₂ as they exerted strong cytokine production upon stimulation through NK1.1 (Figure 3B).

### SUPER NK cells eliminate lung metastases

Deregulated metabolism of arachidonic acid is observed in many human cancers, resulting in increased PGE₂ production due to increased activity of the COX-pathway. Similarly, an increased production of PGE₂ is observed in numerous mouse cancer models, including BRAF^{V600E} cancer cells (Zelenay et al., Cell, 2015; Böttcher et al., Cell, 2018).. This PGE₂-producing cancer model is therefore an ideal system in which to elucidate how PGE₂ signaling switches off NK cell immunosurveillance and to characterize its consequences for NK cell-mediated control of metastasis *in vivo.* To study this, we investigated the formation of lung metastasis following intravenous injection of BRAF ^{V600E} tumor cells that express the fluorescent reporter mCherry, which enabled the detection of metastasis by confocal microscopy. This experimental model allowed us to track metastasis development over time, from early dissemination and seeding within the lung to metastasis outgrowth at later time points.

For the analysis by confocal microscopy, metastatic lungs were isolated and directly placed in Antigenfix (fixation solution) for 4-6 h. After washing the organs with PBS, we generated 300 µm thick vibratome sections which were stained for 2h with Phalloidin.

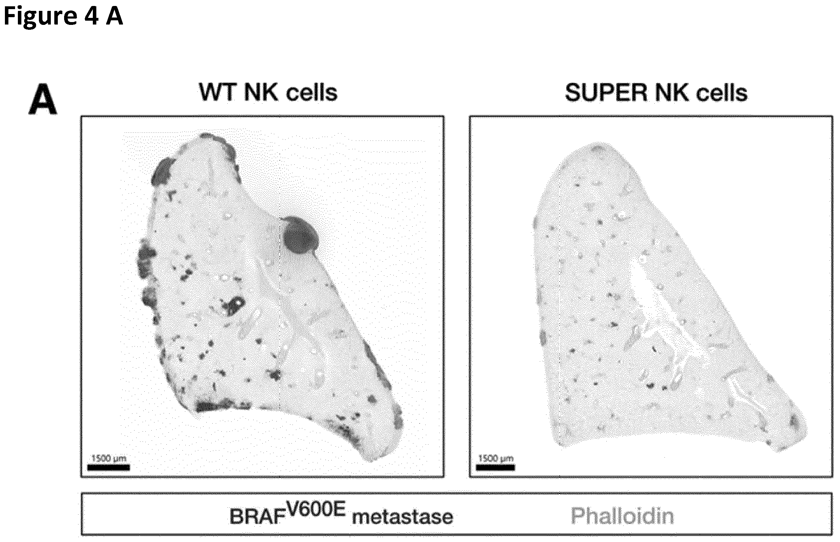

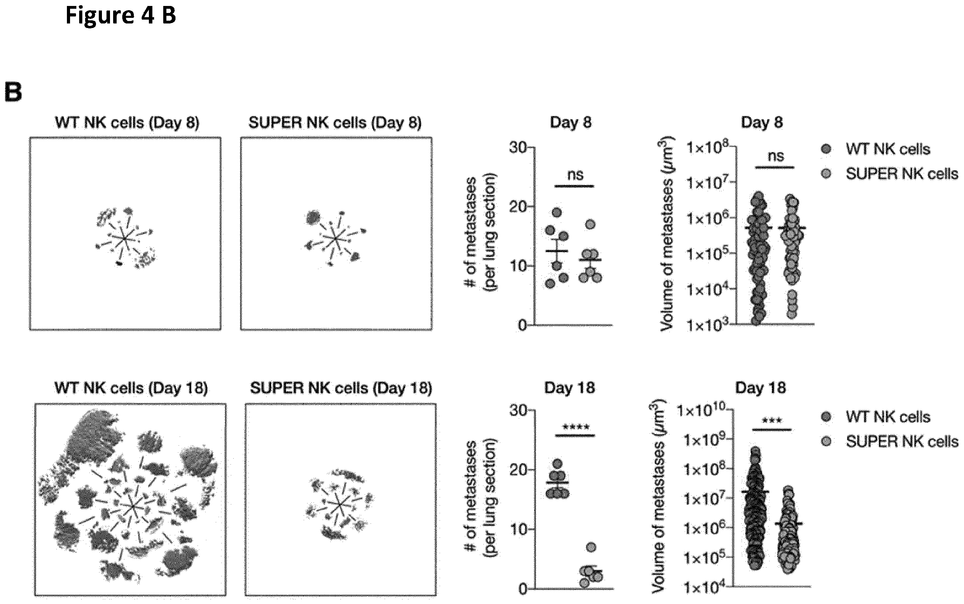

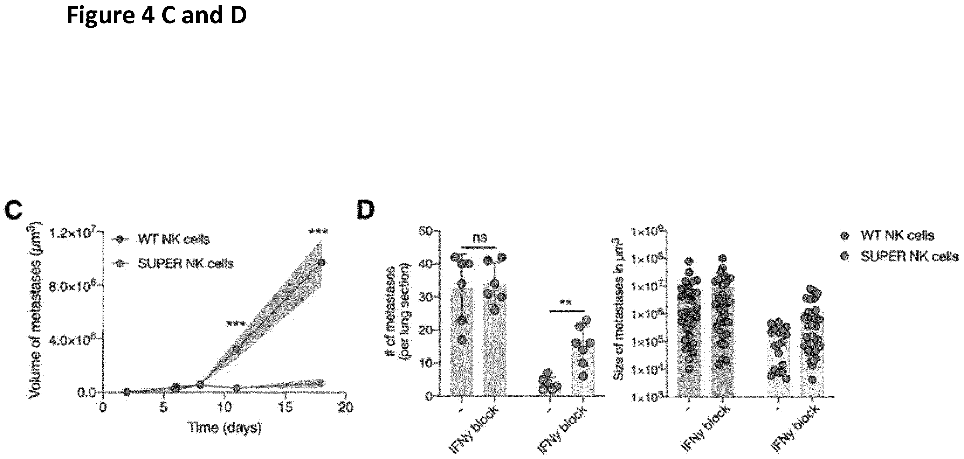

In initial experiments, we compared the metastasis development in WT and GzmB-Cre Ptger2/ Ptger4fl/fl mice 18 days after tumor cell injection. Compared to WT animals, transgenic mice with SUPER NK cells showed an almost complete elimination of lung metastases (Figure 4A). Thus, SUPER NK cells were resistant towards the inactivation by PGE₂ and controlled the metastasis development. The process of metastasis critically depends on the ability of disseminated tumor cells to reside in tissue niches in a dormant state before their reactivation causes outgrowth of metastases. On the one hand, PGE₂ signaling on NK cells could act in the initial phase of metastasis, by preventing NK cells from eliminating disseminated tumor cells. On the other hand, PGE₂-mediated impairment of NK cell function could be critical for metastasis at later stages, for example when dormant tumor cells start to outgrow within lung tissue. To address both options, we compared the metastasis development between WT and GzmB-Cre Ptger2/ Ptger4fl/fl mice over time. Interestingly, there was no difference in number and size of metastases between WT and the transgenic mice on day 8 (Figure 4B, upper panel). But on day 18, there were more and larger metastases in mice with conventional NK cells, whereas mice with SUPER NK cells have less and only small metastases (Figure 4B, lower panel and 4C). Thus, PGE₂ signaling on NK cells is critical for metastasis at later stages and enables the outgrowth of macrometastases.

Based on the results of our *in vitro* experiments, PGE₂ strongly impairs the production of anti-metastatic cytokines including IFNγ. To confirm the relevance of IFNγ production by NK cells in our metastasis model, we blocked this cytokine in WT and GzmB-Cre Ptger2/Ptger4fl/fl mice and analyzed the metastasis development 18 days after tumor cell injection by microscopy. Blocking IFNγ diminished metastasis control in GzmB-Cre Ptger2/ Ptger4fl/fl mice resulting in higher number and size of metastases (Figure 4D). In contrast, neutralization of IFNγ in WT mice had no metastasis-promoting effects compared to the WT control. Thus, IFNγ produced by SUPER NK cells is critical for an efficient elimination of metastases.

### SUPER NK cells can be used to therapeutically control metastatic disease

As demonstrated by the *in vitro* and *in vivo* studies, PGE₂ signaling via EP₂ and EP₄ shuts off NK cell communication pathways and disables immunosurveillance of metastasis. Thus, PGE₂ signaling on NK cells is an attractive target for the treatment of metastatic disease.

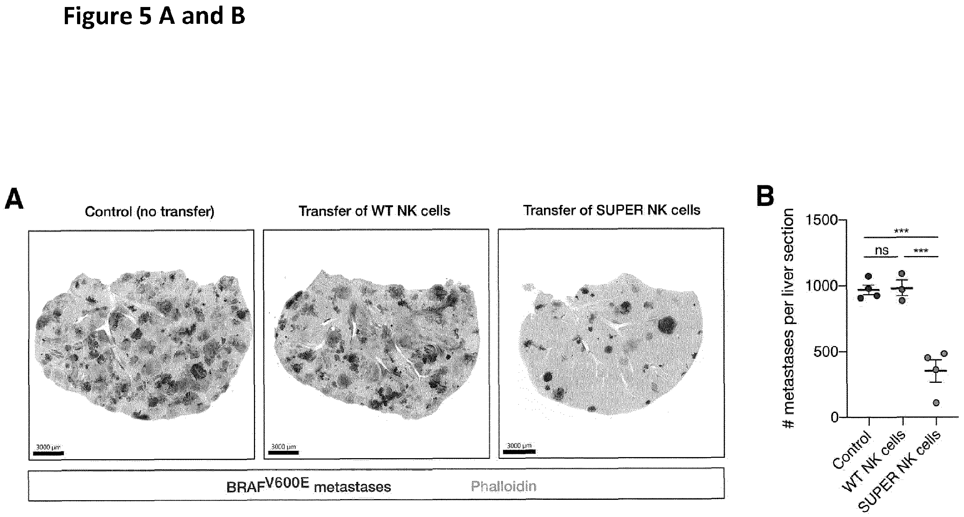

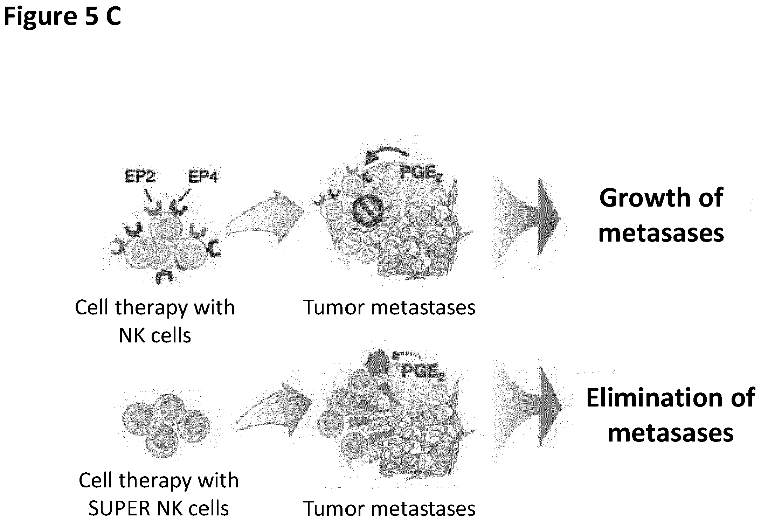

We thus tested the adoptive transfer of SUPER NK cells into mice with established metastases. For the therapeutic approach, we first isolated conventional NK cells and SUPER NK cells from mice and cultured the cells in low doses of IL2 and IL15/15Ra for five days. Meanwhile, we intravenously injected BRAF^{V600E} mCherry tumor cells into immunodeficient Rag2^{-/-}Il2rg^{-/-} mice. Three days after tumor cell injection, we transferred the *in vitro* cultured NK cells. After further 11 days, metastatic livers were isolated and analyzed. Treatment with conventional NK cells did not result in metastasis control. In contrast, the transfer of SUPER NK cells significantly reduced the number of liver metastases (Figure 5A and 5B). This demonstrates that already established metastases respond well to the therapy with SUPER NK cells cells (Figure 5C). Thus, this therapeutic approach can be applied in more advanced diseases stages.

### PGE₂-signaling on NK cells plays a role in human cancer patients

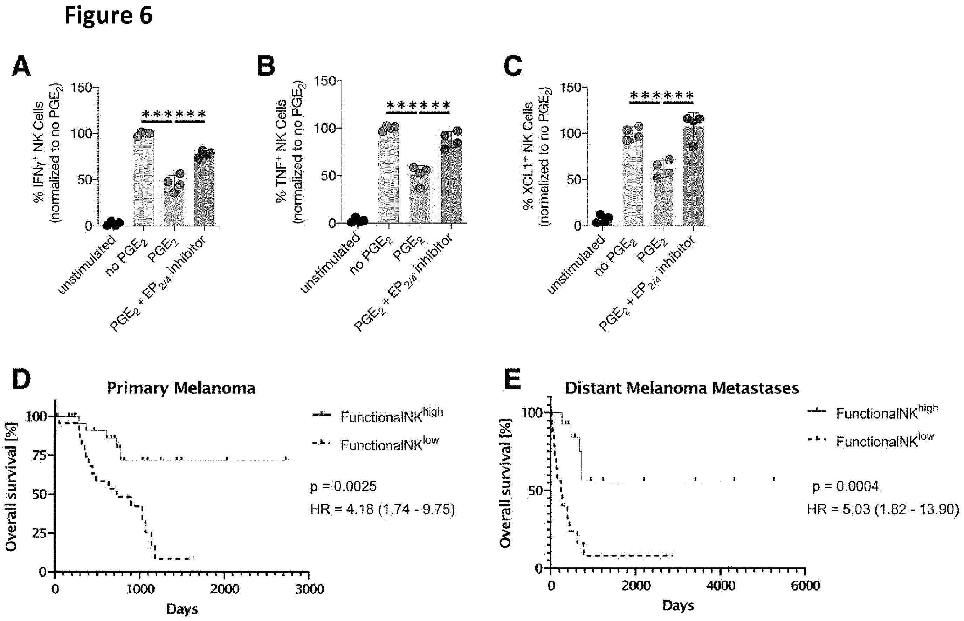

Similar to our mouse cancer model, a deregulated arachidonic acid metabolism leading to increased PGE₂-production is a hallmark of human cancers. Thus, our findings in mice are highly relevant to understand the mechanism that regulate NK cell immunity in primary tumors and metastases in human patients. To find out whether our findings in mice can be translated to the regulation of human NK cells in cancer, we isolated NK cells from human blood. These cells were then pre-treated with EP₂ and EP₄ inhibitors prior to incubation with PGE₂ for 16 h. Cytokine production upon stimulation with K562 was assessed by flowcytometric analysis. We observed that PGE₂ impaired the cytokine and chemokine production by human NK cells, which can be almost completely rescued by the blockade of EP₂ and EP₄ on NK cells (Figure 6A-C).

To investigate the relevance of NK cell abundance and functionality in human cancers, we looked at tumor gene expression data from The Cancer Genome Atlas (TCGA) (TCGA Research Network: https://www.cancer.gov/tcga). We combined NK cell signature genes (NCR3, KLBR1, PRF1, CD 160 and NCR1) with the Core PGE₂ gene set, including IFNγ, TNF, XCL1, identified in the RNA sequencing approach and correlated the gene expression with patient survival. When analyzing TCGA datasets for skin tumor and distant melanoma metastases, we observed a strong correlation between high expression of functional NK signature genes and better patient survival (Figure 6D and 6E). Thus, this gene signature can be used as prognostic factor for patient outcome.

The following examples and drawings illustrate the present invention without, however, limiting the same thereto.

### BRIEF DESCRIPTION OF THE DRAWINGS

**Figure 1****.** *PGE₂ impairs cytokine and chemokine production by NK cells.*
   (A-F) In all *in vitro* assays, cultured conventional NK cells were first pre-treated with PGE₂ for 16 h. (A) Release of cytotoxic granules by degranulation upon anti-NK1.1 stimulation and (B) expression of the cytotoxic effector molecule granzyme B were assessed by flowcytometric analysis. (C) NK cell mediated killing of tumor cells was measured using the XCelligence system. (D-F) Quantification of cytokines, chemokines and growth factors secreted by NK cells upon NK1.1 stimulation was done by ELISA (D-E) or multiplex cytokine assays (F).
**Figure 2****.** *PGE₂ signaling stably imprints a dysfunctional program in NK cells.*
   (A-D) Conventional NK cells were exposed to PGE₂ for 1 h and then stimulated by anti-NK1.1 for additional 4 h before subsequent analysis by RNA sequencing. (A) Schematic illustration of the analysis and filtering process of the sequencing data. (B) Principal component analysis (PCA) depicting differences in global gene expression for conventional NK cells (untreated), conventional NK cells stimulated with anti-NK1.1 (untreated+aNK1.1) and conventional NK cells treated with PGE2 followed by stimulation with anti-NK1.1 (PGE₂+aNK1.1). (C) Pathway analysis on core PGE₂ genes showing the eight most significantly regulated pathways blocked by PGE₂ signaling in conventional NK cells. (D) Heatmap showing protein expression of key cytokines, chemokines and growth factors involved in metastatic activity of NK cells. (E) Effect of constant or transient treatment of conventional NK cells with PGE₂ prior to anti-NK1.1 stimulation on the production of the chemokine XCL1. XCL1 production upon stimulation was quantified in supernatants by ELISA.
   (F, G) Analysis of epigenetic changes induced by PGE2 signaling in conventional NK cells by ATAC sequencing. Conventional NK cells were exposed to PGE₂ for 1 h and then stimulated for further 4 h by anti-NK1.1 prior to the sequencing. (F) Volcano plot showing differentially accessible genomic regions between conventional NK cells (untreated) and conventional NK cells activated by anti-NK1.1 (untreated+aNK1.1). Highlighted are promoter regions of gnees encoding for key NK cell effector molecules that gain genomic accessibility upon stimulation. (G) Volcano plot showing differentially accessible regions between conventional NK cells (untreated) and PGE₂-exposed conventional NK cells activated by anti-NK1.1 (untreated+aNK1.1), highlighting the inability of genomic regions to open upon NK cell stimulation following PGE₂-treatment.
**Figure 3****.** *PGE₂ mediated impairment of cytokine production depends on EP₂ and EP₄.* (A) Conventional NK cells were pre-treated with synthetic inhibitors for the PGE₂-receptors EP₁, EP₂, EP₃ and/or EP₄ and subsequently incubated with PGE₂ for 16 h. IFNγ production upon anti-NK1. 1 stimulation was quantified in supernatants by ELISA. (B) Conventional (WT) or SUPER NK cells (EP2- and EP4-deficient) were exposed to PGE₂ for 16 h and then stimulated with anti-NK1 .1. IFNγ produced by NK cells was quantified by ELISA.
**Figure 4****.** *SUPER NK cells efficiently eliminate lung metastases.*
   (A-D) Development of lung metastases following intravenous (i.v.) injection of metastatic BRAFV600E tumor cells into wildtype mice (WT, harboring conventional NK cells) or transgenic mice in which GzmB-expressing cells such as NK cells are selectively deficient in EP₂ and EP₄ (SUPER NK cells).
   (A) Analysis of lung metastasis by confocal microscopy 18 days after tumor cell injection.
   (B) Visualization and quantification of metastasis volume and numbers in mice with conventional (WT) and SUPER NK cells on day 8 (*upper panel*) and day 18 (*lower panel*) post tumor cell injection.
   (C) Quantification of metastasis development over time.
   (D) Impact of neutralization of the NK cell-derived cytokine IFNγ on metastasis formation. Metastases in mice with conventional (WT) or SUPER NK cells were analyzed 18 days after tumor cell injection.
**Figure 5****.** *Established metastases are eliminated by SUPER NK cells after adoptive transfer.*
   (A-B) Treatment of established liver metastases in Rag2^{-/-}Il2rg^{-/-} mice (lacking endogenous NK cells) with adoptively transferred conventional (WT) or SUPER NK cells. Mice without NK cell transfer served as control. (A) Microscopy analysis of metastases in livers. (B) Quantification of liver metastases.
   (C) Scheme showing the targeted therapy of metastasis with SUPER NK cells.
**Figure 6****.** *PGE₂ impairs human NK cells via EP2*/*EP4 and is associated with poor cancer patient survival.*
   (A-C) Human NK cells were pre-treated with EP₂ and EP₄ inhibitors followed by incubation with PGE₂ for 16 h. Production of the effector molecules (A) IFNγ, (B) TNF and (C) XCL1 upon stimulation with the tumor cell line K562 was assessed by flowcytometric analysis. (D-E) Correlation of a gene signature for functional NK cells with patient survival for (D) skin tumor samples and (E) distant melanoma metastases. The functional NK cell signature contains NK cell-specific genes and genes associated with NK cell function that have been identified to be selectively impaired by PGE₂ signaling in our core PGE₂ RNA-sequencing analysis (NCR3, KLBR1, PRF1, CD160, NCR1, IFNγ, TNF, IL2, XCL1, CCL4, CCL15, CCL3, CCL1, CSF2, IL2Ra, CRTAM, ICAM1, TNFSF9, TNFSF14, TNFSF8).

### EXAMPLES

### EXAMPLE 1 Materials and Methods

### Mice

All mice used in this study were bred and maintained under specific-pathogen□free conditions at the School of Medicine, Technical University Munich (TUM), according to the guidelines of the Federation of Laboratory Animal Science Association. All mice were on a C57BL/6 genetic background. The following strains were used: C57BL/6J wildtype mice (WT, as control), *GzmB^{Cre} EP2*^{*-*/}*⁻ EP4*^{*fl*/*fl*} (in which expression of the Cre-recombinase under the GzmB promoter results in a double knockout of EP4 and EP2 selectively in GzmB-expressing NK cells) and *Rag2*^{*-*/}*⁻IL2r*^{-/*-*} (lacking NK cells and other adaptive lymphocytes). Experiments were conducted using age- and gender-matched mice in accordance with approved institutional protocols. Animals were typically 6-10 weeks old at the time of use and consisted of males and females.

### Cell lines

BRAF^{V600E} melanoma cell lines were cultured in complete RPMI medium (RPMI 1640 with 10% fetal calf serum, 50mM 2-mercaptoethanol, 100U/ml Penicillin, 100mg/ml Streptomycin, 2mM L-Glutamine). Please note that these cells, similar to numerous other tumor cell models, produce large amounts of PGE₂. Cell lines expressing the mCherry reporter were generated by retroviral transduction as described previously (Böttcher *et al*., Cell, 2018).

### Isolation and culture of mouse NK cells

For the isolation of mouse NK cells, the spleen was excised from mice and passed through a steel sieve. Red blood cells were lysed by incubating the cells with ammonium chloride potassium buffer for 2 min. Following filtration through a 70µm cell strainer, CD49b⁺ leukocytes were purified by immunomagnetic separation to enrich for CD49b⁺ NK cells. Th cells were stained with anti-CD49b FITC, anti-CD3ε PerCP/Cy5.5, anti-CD19 PE/eF610 and CD64 PE/Cy7 for 15 min at 4 °C and NK cells, phenotypically identified as CD49b⁺CD3ε⁻CD19⁻CD64⁻ cells, were FACS-sorted using a SH800 Cell Sorter. After isolation, NK cells were cultured in low doses of IL2 and IL15/IL15Ra for five days to rest the cells and generate functional NK cells.

### Isolation of human NK cells from peripheral mononuclear cells

Human NK cells were isolated from blood of healthy, voluntary donors after they gave written and informed consent. Blood was mixed with PBS and loaded on Pancoll to isolate peripheral blood mononuclear cells (PBMCs) through centrifugation. NK cells were then isolated by negative selection using the Untouched Human NK Cell Isolation Kit (ThermoFisher).

### NK cell treatment with PGE₂

Murine or human NK cells were treated with 1 - 100 ng/ml synthetic PGE₂ or medium as control for 16 h at 37 °C. For the inhibitor studies, NK cells were first incubated with single EP receptor inhibitors (EP₁ - EP₄) or inhibitor combinations at a concentration of 5 µM for 30 min, before adding 100 ng/ml PGE₂ for 16 h at 37 °C. For the RNA and ATAC sequencing approach, NK cells were only pre-treated for 1h with PGE₂ and then stimulated (as described below) for further 4h.

### NK cell stimulation and analysis of NK cell effector function by flow cytometry

To assess cytokine and chemokine production and degranulation, NK cells were stimulated with plate-bound anti-NK1.1 (PK136), mimicking their activation in metastasis. Human NK cells were activated by incubation with the human lymphoma cell line K562. For analysis of cytokine and chemokine production, Brefeldin A was added 1h after stimulation to block intracellular protein transport processes. 3h later, cells were stained for flow cytometric analysis. For analysis of degranulation, stimulation was done in presence of anti-LAMP-1 antibody, and Monensin was added 1h later to prevent acidification of endolytic vesicles.

For flow cytometric analysis, cells were resuspended in staining buffer containing antibodies for surface molecules and incubated for 15 min. A fixable viability dye was used to exclude dead cells in all experiments. Cells were fixed using the IC fixation buffer kit (Invitrogen) according to the manufacturer's instructions. Intracellular staining of cytokines (including IFNγ and TNF) was performed in permeabilization buffer and cells were subsequently analyzed by flow cytometry. Staining for intracellular Granzyme B was done using the Foxp3/Transcription factor buffer set from ebioscience.

### Cytokine release assay

To assess the production of cytokines in cell culture supernatants, NK cells were stimulated for 16 h and cell-free supernatants were harvested and stored at -20 °C until further use. Cytokine concentrations in the culture supernatants were measured by enzyme-linked immunosorbent assays (ELISA) or a multiplex cytokine assay (Bio-Plex Pro Mouse Cytokine 23-plex).

### NK cell cytotoxicity assay

Measurement of the dynamics of NK cell cytotoxicity against BRAFV600E tumor cells over time was performed with the impedance □ based technology using an xCelligence RTCA MP device (ACEA Biosciences). The xCELLigence RTCA system measures changes in impedance generated by adherence of cells to electrodes on the bottom of the wells of E-Plates and calculates a parameter called Cell Index. For the assays, tumor cells were seeded in 96-well E-plates. After tumor cells reached confluency and a maximum cell index, *in vitro* cultured NK cells were added in different effector-to-target ratios. Killing was measured by declining cell index values. Percentage of specific cytotoxicity was finally calculated to the index values from tumor cells alone.

### RNA isolation and quantitative real-time PCR

RNA from NK cells was isolated using Monarch Total RNA Miniprep Kit and cDNA was generated using SensiFAST cDNA synthesis kit from Bioline. Primers against human XCL1/2 and HPRT were purchased from Eurofins. A mix of primer pairs, double-distilled water and 2× Takyon Mix SYBR green assay was added to the cDNA and amplified copies were quantified by LightCycler 480 (Roche).

### RNA-sequencing

RNA was isolated from sorted or stimulated NK cell population using Monarch Total RNA Miniprep Kit. Following quality control by Agilent Bioanalyzer, RNA was subjected to cDNA synthesis. Barcoded cDNA of each sample was produced with a Maxima RT polymerase (Thermo Fisher) exercising oligo-dT primer containing barcodes, unique molecular identifiers (UMIs) and an adaptor. 5' ends of the cDNAs were prolonged by a template switch oligo (TSO). After pooling all samples, full-length cDNA was amplified with primers binding to the TSO site and the adaptor. cDNA was supplemented with the Nextera XT kit (Illumina) and 3'-end-fragments finally amplified using P5 and P7 Illumina overhangs. The barcoded cDNA libraries were sequenced using a NextSeq 500 (Illumina).

### ATAC-sequencing

ATAC Sequencing was performed as described previously (Buenrostro et al., Nature Methods, 2013). Briefly, NK cells were washed in cold PBS and lysed. The transposition reaction was incubated at 37 °C for 30 min. DNA was purified using the Monarch PCR & DNA Cleanup Kit and material was amplified for four cycles. After evaluation by real-time PCR, cycles were added as calculated. The amplified samples were purified and size selected using SpeedBeads. Libraries were controlled by BioAnalyzer and sequenced on a NovaSeq 6000 (Illumina).

### Bioinformatic analysis of cancer patient data

Gene expression datasets from The Cancer Genome Atlas (TCGA) were downloaded from Firehose (https://gdac. broadinstitute.org/). Gene signatures for functional NK cells contained a combination of NK cell-specific gene and genes identified to be suppressed by PGE2-signaling in our core PGE₂ gene expression dataset (NCR3, KLBR1, PRF1, CD160, NCR1, IFNγ, TNF, IL2, XCL1, CCL4, CCL15, CCL3, CCL1, CSF2, IL2Ra, CRTAM, ICAM1, TNFSF9, TNFSF14, TNFSF8). Overall survival analyses were performed for the top and bottom quartile ranked sum expression of signature genes and plotted for Kaplan-Meier curves using GraphPad Prism (GraphPad).

### Tumor cell injection for metastasis development

Tumor cells were harvested by trypsinization and washed three times in PBS. 1×10⁶ cells were intravenously injected in 100µl endotoxin-free PBS to induce development of lung metastases.

### Analysis of lung metastases by immunofluorescence microscopy

Mice were anaesthetized in a chamber containing 45% isoflurane. Under deep anesthesia, mice were perfused with 10ml PBS via the right ventricle of the heart to eliminate vascular blood content. In a second step, perfusion was continued with 10ml Antigenfix solution (Diapath) to enable maximal epitope conservation. Pre-warmed 2% low melting point (LMP) agarose was gently injected into the trachea until lungs are inflated to full capacity. Organs were isolated and fixed for 4-6h in Antigenfix. Following tissue fixation, lungs were embedded into melted 4% LMP agarose. Once solidified, agar tissue blocks were sectioned (300µm thickness) using a VT1000S vibratome. Lung sections were permeabilized, blocked and stained in 0.1M Tris supplemented with 1% BSA, 0.3% Triton X-100 (Gerbu Biotechnik) and normal mouse serum. For staining actin filaments, tissue was incubated with Phalloidin for 2h. Stained sections were mounted in Mowiol and analyzed on a confocal microscope with motorized stage (Zeiss) or a THUNDER imager (Zeiss). Image analysis was performed using Imaris software (Bitplane) on maximum projections of 11 Z-plane sections (20µm thickness). Semi-automated analyses using the Imaris surface generation tool was used to reconstruct surfaces for mCherry⁺ metastases.

### Cytokine neutralization in vivo

For neutralization of IFNγ, 400mg of anti-IFNy antibody was injected intraperitonally (i.p.) two days after tumor cell injection. Antibody injections were performed every four days during the course of the experiment.

### Adoptive transfer of NK cells into Rag2^{-/}⁻ IL2rg^{-/-}

Conventional NK cells or SUPER NK cells (derived from *GzmB^{Cre} EP2*^{*-*/}*⁻ EP4*^{*fl*/*fl*} mice) were isolated from spleen and cultured in low doses of IL2 and IL15/15Ra for five days. Meanwhile, BRAF ^{V600E} mCherry tumor cells were intravenously injected into immunodeficient *Rag2*^{*-*/*-*} *Il2rg*^{*-*/*-*} mice. Three days after tumor cell injection, *in vitro* cultured NK cells were washed and adoptively transferred into mice. After further 11 days, metastatic livers were isolated and analyzed by immunofluorescence microscopy.

The features disclosed in the foregoing description, in the claims and/or in the accompanying drawings may, both separately and in any combination thereof, be material for realizing the invention in diverse forms thereof.

### REFERENCES

Bald, T., M. F. Krummel, M. J. Smyth, and K. C. Barry. 2020. The NK cell-cancer cycle: advances and new challenges in NK cell-based immunotherapies. Nature Immunology 21: 1-13.
Böttcher JP, Bonavita E, Chakravarty P, Blees H, Cabeza-Cabrerizo M, Sammicheli S, Rogers NC, Sahai E, Zelenay S, Reis e Sousa C. NK Cells Stimulate Recruitment of cDCl into the Tumor Microenvironment Promoting Cancer Immune Control. Cell. 2018;172(5):1022-1037. e14. doi: 10.1016/j.cell.2018.01.004.
Bonavita E, Bromley CP, Jonsson G, Pelly VS, Sahoo S, Walwyn-Brown K, Mensurado S, Moeini A, Flanagan E, Bell CR, Chiang SC, Chikkanna-Gowda CP, Rogers N, Silva-Santos B, Jaillon S, Mantovani A, Reis e Sousa C, Guerra N, Davis DM, Zelenay S. Antagonistic Inflammatory Phenotypes Dictate Tumor Fate and Response to Immune Checkpoint Blockade. Immunity. 2020;53(6): 1215-1229. e8. doi: 10.1016/j.immuni.2020.10.020.
Buenrostro, J. D., Giresi, P. G., Zaba, L. C., Chang, H. Y. & Greenleaf, W. J. Transposition of native chromatin for fast and sensitive epigenomic profiling of open chromatin, DNA-binding proteins and nucleosome position. Nat. Methods 10, 1213-1218 (2013).
Burn, J., et al., Cancer prevention with aspirin in hereditary colorectal cancer (Lynch syndrome), 10-year follow-up and registry-based 20-year data in the CAPP2 study: a double-blind, randomised, placebo-controlled trial. Lancet, 2020; 395(10240): p. 1855-1863.
Glasner, A., A. Levi, J. Enk, B. Isaacson, S. Viukov, S. Orlanski, A. Scope, T. Neuman, C. D. Enk, J. H. Hanna, V. Sexl, S. Jonjic, B. Seliger, L. Zitvogel, and O. Mandelboim. 2018. NKp46 Receptor-Mediated Interferon-y Production by Natural Killer Cells Increases Fibronectin 1 to Alter Tumor Architecture and Control Metastasis. Immunity 48: 107-119.e4.
Guillerey, C., N. D. Huntington, and M. J. Smyth. 2016. Targeting natural killer cells in cancer immunotherapy. Nat. Immunol. 17: 1025-1036.
Guo, Q., et al., A comprehensive evaluation of clinical efficacy and safety of celecoxib in combination with chemotherapy in metastatic or postoperative recurrent gastric cancer patients: A preliminary, three-center, clinical trial study. Medicine (Baltimore), 2019; 98(27): p. e16234.
Lorenzo-Herrero, S., A. Lopez-Soto, C. Sordo-Bahamonde, A. Gonzalez-Rodriguez, M. Vitale, and S. Gonzalez. 2019. NK Cell-Based Immunotherapy in Cancer Metastasis. Cancers (Basel) 11: 29-22.
Morvan, M. G., and L. L. Lanier. 2016. NK cells and cancer: you can teach innate cells new tricks. Nature Reviews Cancer 16: 7-19.
Wang, D., and R. N. DuBois. 2016. The Role of Prostaglandin E2 in Tumor-Associated Immunosuppression. Trends Mol Med 22: 1-3.
Zelenay, S., A. G. Van der Veen, J. P. Böttcher, K. J. Snelgrove, N. Rogers, S. E. Acton, P. Chakravarty, M. R. Girotti, R. Marais, S. A. Quezada, E. Sahai, and C. Reis e Sousa. 2015. Cyclooxygenase-Dependent Tumor Growth through Evasion of Immunity. Cell 162: 1257-1270.

## Claims

1. A modified natural killer (NK) cell or T cell,
wherein the expression and/or activity of prostaglandin E receptor 2 (EP2) and of prostaglandin E receptor 4 (EP4) is selectively inhibited or eliminated.

2. The modified NK or T cell of claim 1, wherein the expression of prostaglandin E receptor 2 (EP2) and of prostaglandin E receptor 4 (EP4) is selectively inhibited or eliminated by genetic modification.

3. The modified NK or T cell of claim 1 or 2,
- which is genetically modified by knocking out EP2 and EP4,
preferably by using the CRISPR/Cas9 gene editing system, or
- which is modified or edited by knocking out EP2 and EP4,
preferably by using zinc-finger nucleases (ZFNs), transcription activator-like effector nucleases (TALENs) and MegaTAL nucleases, or by using base editing, or
- which is modified by gene silencing/knockdown of EP2 and EP4,
preferably via introduction of short-hairpin RNAs via the Sleeping Beauty transposon system, lentiviral vectors or gamma-retroviral vectors.

4. The modified NK or T cell of any one of claims 1 to 3, which is further modified by introducing component(s), such as a chimeric antigen receptor (CAR), a chemokine or cytokine receptor; an activating receptor; or by elimination of other genes, such as other inhibitory receptors.

5. The modified NK or T cell of any one of claims 1 to 4 for use in the treatment and/or prevention of metastatic disease and cancer,
wherein the cancer is preferably a solid tumor, such as a primary or secondary solid tumor, a hematological tumor, and/or metastases,
wherein, preferably, the treatment and/or prevention of cancer comprises adoptive immune cell therapy, more preferably adoptive NK cell therapy, adoptive T cell therapy, CAR T cell therapy, CAR NK cell therapy and combinations thereof.

6. The modified NK or T cell for use according to claim 4 or 5, comprising an adjuvant therapy, such as after surgery of the primary tumor.
and/or comprising treatment of metastasis, including advanced metastasis.

7. The modified NK or T cell for use according to any one of claims 4 to 6, comprising a combination therapy with other immune therapies, such as checkpoint blockade inhibitors targeting PD-1, CTLA-4, and/or PD-L1, and/or a combination with anti-programmed death (PD) protein 1 therapy, and/or combinations thereof.

8. A method for generating a modified NK or T cell according to any one of claims 1 to 3, comprising the following steps:
(a) providing leukocytes, preferably lymphocytes, from a healthy subject/donor or from a patient,
(b) separating the NK or T cells and *in vitro* expanding the NK or T cells;
(c) genetically modifying the NK or T cells in order to selectively eliminate the expression of EP2 and EP4;
preferably by knocking out EP2 and EP4 or by gene silencing/knockdown of EP2 and EP4, and
(d) *in vitro* expanding the genetically modified NK or T cells ;
(e) optional, cryopreserving the genetically modified NK or T cells obtained in step (d).

9. The method of claim 8, wherein in step (a) the leukocytes, preferably lymphocytes, are
from periphery blood, preferably obtained via leukapheresis,
from cord blood, such as from a cord blood bank, or
from induced pluripotent stem cells,
and/or wherein the leukocytes, preferably lymphocytes, are from autologous cells derived from a patient or are allogeneic cells derived from an unrelated healthy subject/donor.

10. The method of claim 8 or 9, wherein in step (c) the genetic modification is carried out by
using the CRISPR/Cas9 gene editing system, or
using zinc-finger nucleases (ZFNs), transcription activator-like effector nucleases (TALENs) and MegaTAL nucleases, or by using base editing, or
gene silencing/knockdown via introduction of short-hairpin RNAs via the Sleeping Beauty transposon system, lentiviral vectors or gamma-retroviral vectors.

11. The method of any one of claims 8 to 10, comprising an additional modification of the NK cell or T cell, such as by introducing component(s), e.g. a CAR, a chemokine or cytokine receptor; an activating receptor; or by elimination of other genes, such as other inhibitory receptors.

12. A method for the treatment and/or prevention of cancer, preferably for the treatment and/or prevention of metastasis, comprising the step of
administering to a subject in need thereof modified NK and/or T cells,
wherein the expression and/or activity of prostaglandin E receptor 2 (EP2) and of prostaglandin E receptor 4 (EP4) is selectively inhibited or eliminated,
preferably administering modified NK and/or T cells according to any one of claims 1 to 3 or modified NK and/or T cells obtained by a method of any one of claims 8 to 11.

13. The method of claim 12, comprising the following steps:
(a) providing leukocytes, preferably lymphocytes, from a healthy subject/donor or from a patient,
(b) separating the NK or T cells and *in vitro* expanding the NK or T cells;
(c) genetically modifying the NK cells in order to selectively eliminate the expression of EP2 and EP4;
preferably by knocking out EP2 and EP4 or by gene silencing/knockdown of EP2 and EP4,
(d) *in vitro* expanding the genetically modified NK or T cells ;
(e) optional, cryopreserving the genetically modified NK or T cells obtained in step (d);
(f) administering the genetically modified NK and/or T cells obtained in step (d) or from step (e), preferably via infusion,
wherein, preferably, in step (a) the leukocytes, preferably lymphocytes, are
from periphery blood, preferably obtained via leukapheresis,
from cord blood, such as from a cord blood bank, or
from induced pluripotent stem cells,
and/or wherein the leukocytes, preferably lymphocytes, are from autologous cells derived from a patient or are allogeneic cells derived from an unrelated healthy subject/donor,
and/or wherein, preferably, in step (c) the genetic modification is carried out by
using the CRISPR/Cas9 gene editing system, or
using zinc-finger nucleases (ZFNs), transcription activator-like effector nucleases (TALENs) and MegaTAL nucleases, or by using base editing, or
gene silencing/knockdown via introduction of short-hairpin RNAs via the Sleeping Beauty transposon system, lentiviral vectors or gamma-retroviral vectors.

14. A composition comprising
(a) a modified NK and/or T cell of any one of claims 1 to 3,
which are preferably obtained by a method of any one of claims 9 to 11,
(b) optional, excipient(s) and/or carrier.

15. The composition of claim 14, wherein the modified NK or T cells are cryopreserved.
